# EUROPÄISCHE PATENTSCHRIFT

(11) **EP 3 774 719 B1**
(45) Veröffentlichungstag und Bekanntmachung des Hinweises auf die Patenterteilung: **23.07.2025**
(21) Anmeldenummer: 19715091.5
(22) Anmeldetag: 03.04.2019
(51) Int. Cl.: C07C 217/06, C07C 51/08, C07C 57/30, C07C 253/14, C07C 255/35, C07C 47/56, C07C 29/141, C07C 33/46, C07C 309/66, C07C 309/73, C07C 45/51, C07F 3/02

(54) **VERFAHREN ZUR HERSTELLUNG VON 2,6-DIALKYLPHENYL-ESSIGSÄUREN**
METHOD FOR THE PREPARATION OF 2.6-DIALKYLPHENYL ACETIC ACIDS
PROCÉDÉ DE PRODUCTION D'ACIDES 2,6-DIALKYLPHÉNYL ACÉTIQUES

(30) Priorität: 10.04.2018 EP 18166472
(43) Veröffentlichungstag der Anmeldung: 17.02.2021
(73) Patentinhaber: Bayer Aktiengesellschaft, 51373 Leverkusen (DE)
(72) Erfinder: HIMMLER, Thomas, 51519 Odenthal (DE); BROHM, Dirk, 40822 Mettmann (DE); MORADI, Wahed, Ahmed, 40789 Monheim (DE)
(74) Vertreter: BIP Patents
(86) Internationale Anmeldenummer: PCT/EP2019/058356
(87) Internationale Veröffentlichungsnummer: WO 2019/197232

(56) Entgegenhaltungen:
- WO-A1-2009/045479
- WO-A1-2013/025425
- WO-A1-2017/121699
- WO-A2-2016/071920
- DATABASE REGISTRY CHEMICAL ABSTRACTS SERVICE, COLUMBUS, OHIO, US; 4 March 2016 (2016-03-04), UKRORGSYNTEZ LTD, XP002783880, Database accession no. 1879859-20-7
- DATABASE REGISTRY CHEMICAL ABSTRACTS SERVICE, COLUMBUS, OHIO, US; 26 February 2016 (2016-02-26), UKRORGSYNTEZ LTD, XP002783881, Database accession no. 1874998-32-9
- DATABASE REGISTRY CHEMICAL ABSTRACTS SERVICE, COLUMBUS, OHIO, US; 9 May 2014 (2014-05-09), UKRORGSYNTEZ LTD, XP002783882, Database accession no. 1601046-43-8
- G.R. GEIER ET AL,: "Effects of aldehyde or dipyrromethane substituents on the reaction course leading to meso-substituted porphyrins", TETRAHEDRON, vol. 60, no. 50, 18 October 2004 (2004-10-18), pages 11435 - 11444, XP004628604, DOI: 10.1016/j.tet.2004.09.081
- G.R. GEIER ET AL: "Supplementary Data to Effects of aldehyde or dipyrromethane substituents on the reaction course leading to meso-substituted porphyrins", TETRAHEDRON, vol. 60, no. 50, 18 October 2004 (2004-10-18), pages S1 - S29, XP002783883, DOI: 10.1016/j.tet.2004.09.081
- XIANG L ET AL: "Stereoselective Synthesis of All Individual Isomers of @b-Methyl-2@?,6@?-dimethylphenylalanine", TETRAHEDRON ASYMMETRY, vol. 6, no. 1, 1 January 1995 (1995-01-01), PERGAMON PRESS LTD, OXFORD, GB, pages 83 - 86, XP004048494, ISSN: 0957-4166, DOI: 10.1016/0957-4166(94)00357-H
- LÖFGREN NILS ET AL: "Syntheses of three Xylocaine(R) Analogues. Steric Effects in the Reaction between 2,6-Dimethylphenyllithium and Epichlorohydrin", ACTA CHEMICA SCANDINAV, vol. 17, 1 January 1963 (1963-01-01), MUNKSGAARD, COPENHAGEN, DK, pages 1252 - 1261, XP009127586, ISSN: 0904-213X, DOI: 10.3891/ACTA.CHEM.SCAND.17-1252

## Beschreibung

Die vorliegende Erfindung betrifft ein Verfahren zur Herstellung von 2,6-Dialkylphenyl-essigsäuren der allgemeinen Formel (I).

2,6-Dialkylphenyl-essigsäuren der allgemeinen Formel (I) sind wichtige Intermediate für die Herstellung bioaktiver Verbindungen, die speziell zur Kontrolle von Schädlingen im Pflanzenschutz eingesetzt werden können. Insbesondere dienen sie zur Herstellung insektizider, akarizider oder herbizider cyclischer Ketoenole (beispielsweise WO 2006/089633*),* wobei aus diesen 2,6-Dialkylphenyl-essigsäuren die entsprechenden 2,6-Dialkylphenyl-essigsäurechloride hergestellt werden.

Es sind bereits verschiedene Methoden zur Herstellung von 2,6-Dialkylphenyl-essigsäuren bekannt geworden (beispielsweise Bioorg.&Med.Chem. 17 (2009) 4241-56*;* Chem.Eur.J. 19 (2013) 7334-7*;* WO 2004/050607*;* WO 2010/104217*;* US 20110039701*;* WO 2011/003530*;* WO 2011/089072*;* WO 2018/015489*).* Diese Methoden sind jedoch nicht in allen Belangen befriedigend. So sind beispielsweise bei einigen dieser Methoden die Ausbeuten unbefriedigend oder es müssen teure Reagenzien wie beispielsweise Palladiumkatalysatoren verwendet werden, wodurch die Synthese unökonomisch werden kann. Zudem ist es bei der Herstellung von Wirkstoffen für den Pharma- oder Agrobereich erforderlich, das Palladium oder andere Schwermetalle bis auf sehr geringe tolerierte Restmengen zu entfernen. Die Verwendung empfindlicher Übergangsmetallkatalysatoren erfordert außerdem den Einsatz sehr sauberer Ausgangsmaterialien, da es anderenfalls leicht zu einer Deaktivierung ("Vergiftung") des Katalysators kommen kann.

Eine Möglichkeit zur Herstellung von 2,6-Dialkylphenyl-essigsäuren ohne die Verwendung eines Palladiumkatalysators besteht beispielsweise darin, ausgehend von einem 2,6-Dialkyl-brombenzol zunächst einen 2,6-Dialkyl-benzaldehyd zu synthetisieren; diesen Aldehyd zu dem entsprechenden 2,6-Dialkyl-benzylalkohol zu hydrieren; diesen Benzylalkohol in ein entsprechendes 2,6-Dialkylbenzylhalogenid zu überführen; das Benzylhalogenid mit einem anorganischen Cyanid zu dem entsprechenden 2,6-Dialkylphenyl-acetonitril umzusetzen; und abschließend das 2,6-Dialkylphenyl-acetonitril zur 2,6-Dialkylphenyl-essigsäure zu hydrolysieren.

Es ist bereits bekannt geworden *(*WO 2001/23387*),* die Zwischenverbindung (4-Chlor-2,6-dimethylphenyl)-acetonitril herzustellen, indem man 4-Chlor-2,6-dimethyl-brombenzol zunächst mit Butyllithium und dann mit N,N-Dimethylformamid umsetzt und so den 4-Chlor-2,6-dimethyl-benzaldehyd erhält; diesen Aldehyd mittels Natriumborhydrid zu 4-Chlor-2,6-dimethyl-benzylalkohol reduziert; diesen Alkohol mit Thionylchlorid zu 4-Chlor-2,6-dimethyl-benzylchlorid umsetzt; und schließlich dieses Benzylchlorid mit Natriumcyanid in (4-Chlor-2,6-dimethylphenyl)-acetonitril überführt.

Nachteilig an diesem Verfahren sind allerdings die Verwendung von Butyllithium und Natriumborhydrid, die beide teuer und im technischen Maßstab nur schwierig handzuhaben sind (beispielsweise tiefe Reaktionstemperaturen von bis zu -100°C bei der Reaktion mit Butyllithium; Chem.Eur.J. 2013,19, 7334-7).

Es ist auch bereits bekannt geworden, anstelle von Butyllithium Magnesium einzusetzen und auf diese Weise die entsprechenden 2,6-Dialkylphenyl-Grignard-Verbindungen herzustellen (beispielsweise Chem.Eur.J. 2014,20, 6268-71*;* J.Med.Chem. 2017, 60, 1325-42*).* Weiterführende Umsetzungen zu 2,6-Dialkylphenyl-essigsäuren sind darin jedoch nicht offenbart.

Es ist ebenfalls bereits bekannt geworden *(*US 20110039701*;* WO 2010/104217*),* 2,6-Dialkylphenyl-Grignard-Verbindungen mit Paraformaldehyd umzusetzen, um auf diese Weise direkt die entsprechenden Benzylalkohole und daraus die entsprechenden Benzylchloride herzustellen. Nachteilig an diesem Verfahren ist allerdings der Umstand, dass sich die so hergestellten Benzylalkohole nur durch sehr aufwendige Maßnahmen vollständig von Paraformaldehyd befreien lassen. Dies ist aber zwingend notwendig, um im folgenden Schritt die Bildung von stark krebserregendem Bis-(chlormethyl)-ether zu vermeiden.

Es besteht dementsprechend weiterhin Bedarf an einem verbesserten Verfahren zur Herstellung von 2,6-Dialkylphenyl-essigsäuren.

Die vorliegende Erfindung beinhaltet daher ein neues Verfahren zur Herstellung von 2,6-Dialkylphenyl-essigsäuren der Formel (I) 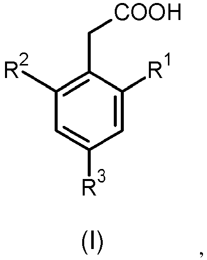 in welcher
- R¹ und R²: für Methyl stehen
und
- R³: für Chlor steht,
dadurch gekennzeichnet, dass in einem ersten Schritt (1) ein 2,6-Dialkyl-brombenzol der Formel (II) 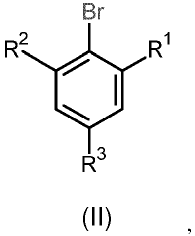
in der R¹, R² und R³ die oben angegebenen Bedeutungen haben,
in Gegenwart eines Lösungsmittels mit Magnesium zu einer Grignard-Verbindung der allgemeinen Formel (III) umgesetzt wird, 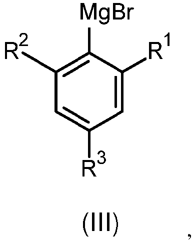
in der R¹, R² und R³ die oben angegebenen Bedeutungen haben;
in einem zweiten Schritt (2) diese Grignard-Verbindung der Formel (III) mit einem N,N-Dialkylformamid der allgemeinen Formel (IV) 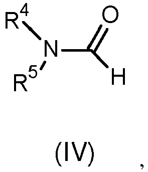
in der
   - R⁴ und R⁵: für Methyl stehen,
zu einer Verbindung der allgemeine Formel (V) umgesetzt wird 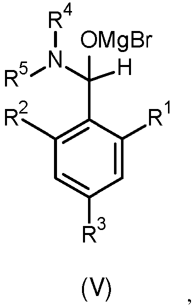
in der R¹, R², R³, R⁴ und R⁵ die oben angegebenen Bedeutungen haben;
in einem dritten Schritt (3) die Verbindung der allgemeinen Formel (V) durch Hydrolyse unter sauren Bedingungen zu einem Aldehyd der allgemeinen Formel (VI) 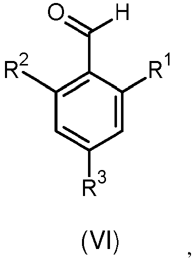
in der R¹, R² und R³ die oben angegebenen Bedeutungen haben, umgesetzt wird;
in einem vierten Schritt (4) der Aldehyd der allgemeinen Formel (VI) zu einem Benzylalkohol der allgemeinen Formel (VII) 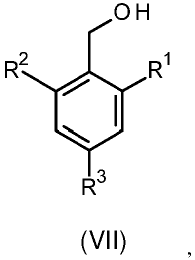
in der R¹, R² und R³ die oben angegebenen Bedeutungen haben, in Gegenwart eines Katalysators hydriert wird;
in einem fünften Schritt (5) der Benzylalkohol der allgemeinen Formel (VII) zu einer Verbindung der allgemeinen Formel (VIII) 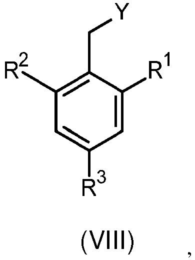
in der R¹, R² und R³ die oben angegebenen Bedeutungen haben und
   - Y: für Chlor oder Brom steht,
umgesetzt wird;
in einem sechsten Schritt (6) die Verbindung der allgemeinen Formel (VIII) mit einem Cyanid der allgemeinen Formel (IX)

   MCN (IX),

   in der
   - M: für Natrium steht,
zu einer Verbindung der allgemeinen Formel (X) 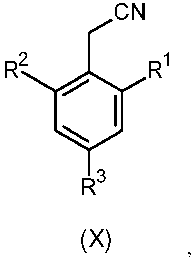
in der R¹, R² und R³ die oben angegebenen Bedeutungen haben, umgesetzt wird;
in einem siebten Schritt (7) die Verbindung der allgemeinen Formel (X) unter sauren oder basischen Bedingungen zu einer 2,6-Dialkylphenyl-essigsäure der allgemeinen Formel (I) 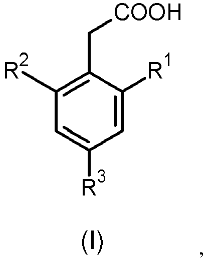
in der R¹, R² und R³ die oben angegebenen Bedeutungen haben, hydrolysiert wird.

Das erfindungsgemäße Verfahren wird durch das Schema 1 veranschaulicht.

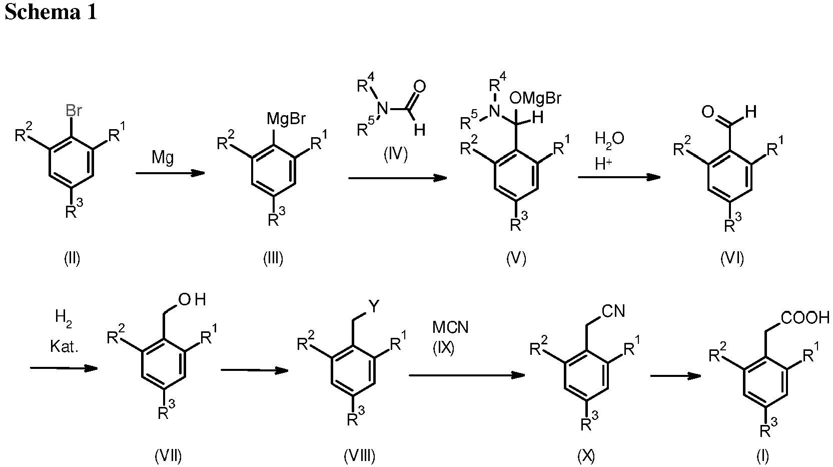

Die Herstellung von 4-Chlor-2,6-dimethylphenylessigsäure wird auch in WO 2017/121699 beschrieben.

Ebenfalls Gegenstand der vorliegenden Erfindung sind neue Verbindungen der allgemeinen Formel (V) 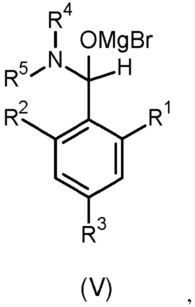 in der R¹, R², R³, R⁴ und R⁵ die oben angegebenen Bedeutungen haben.

In den Formeln (I), (II), (III), (IV), (V), (VI), (VII), (VIII), (IX) und (X) haben R¹, R², R³, R⁴, R⁵, Y und M die oben angegebenen Bedeutungen.

Die Verbindungen der Formeln (II), (III), (IV), (VI), (VII), (IX) und (X) sind entweder kommerziell erhältlich oder lassen sich nach bekannten Verfahren herstellen.

### Allgemeine Beschreibung des erfindungsgemäßen Verfahrens:

### Erster Schritt (1) des erfindungsgemäßen Verfahrens:

Als Lösungs- und Verdünnungsmittel im ersten Schritt des erfindungsgemäßen Verfahrens kommen beispielsweise in Frage: Methyl-tert-butyl-ether, Cyclopentyl-methyl-ether, Tert-Amyl-methylether, 1,2-Dimethoxyethan, Diethylenglykol-diethylether, Tetrahydrofuran, 2-Methyl-tetrahydrofuran, 1,4-Dioxan, Toluol, o-Xylol, m-Xylol, p-Xylol oder Mesitylen oder Mischungen dieser Lösungs- und Verdünnungsmittel.

Bevorzugt als Lösungs- und Verdünnungsmittel sind Methyl-tert-butyl-ether, Cyclopentyl-methyl-ether, Tetrahydrofuran, 2-Methyl-tetrahydrofuran oder Toluol oder Mischungen dieser Lösungs- und Verdünnungsmittel.

Besonders bevorzugt sind Mischungen aus Tetrahydrofuran und Toluol.

Die Menge an Magnesium bezogen auf den Bromaromaten der allgemeinen Formel (II) liegt zwischen 0,9 und 1,5 Mol pro Mol; bevorzugt zwischen 1,0 und 1,3 Mol pro Mol.

Die Reaktion zwischen dem Magnesium und dem Bromaromaten der allgemeinen Formel (II) zur Herstellung der Grignard-Verbindung der allgemeinen Formel (III) kann auf verschiedene, grundsätzlich bekannte Weisen, gestartet werden, beispielsweise durch Zugabe von substöchiometrischen Mengen Iod, Methyliodid, Ethyliodid, 1,2-Dibromethan, Trimethylsilylchlorid, Lösungen von Methylmagnesiumchlorid, Methylmagnesiumbromid, Ethylmagnesiumchlorid, Ethylmagnesiumbromid oder bereits vorhandener Lösung der Grignard-Verbindung der allgemeinen Formel (III). Bevorzugt verwendet man Iod, 1,2-Dibromethan, Ethylmagnesiumbromid und bereits vorhandene Lösung der Grignard-Verbindung der allgemeinen Formel (III). Besonders bevorzugt verwendet man Ethylmagnesiumbromid und bereits vorhandene Lösung der Grignard-Verbindung der allgemeinen Formel (III).

Die Reaktionstemperatur beim ersten Schritt (1) des erfindungsgemäßen Verfahrens liegt zwischen 10 und 70°C, bevorzugt zwischen 10 und 40°C.

Das Produkt des ersten Schrittes wird nicht isoliert sondern als Lösung in den zweiten Schritt des erfindungsgemäßen Verfahrens eingesetzt.

### Zweiter Schritt (2) des erfindungsgemäßen Verfahrens:

Die Grignard-Verbindung der allgemeinen Formel (III) wird mit einem Formamid der allgemeinen Formel (IV) wie beispielsweise N,N-Dimethylformamid, N,N-Diethylformamid, N,N-Dibutylformamid, N-Formyl-piperidin, N-Formyl-morpholin oder N-Formyl-thiomorpholin umgesetzt. Bevorzugt verwendet am N,N-Dimethylformamid, N,N-Dibutylformamid oder N-Formyl-morpholin.

Die Menge an Formamid der allgemeinen Formel (IV) liegt zwischen 0,9 und 2 Mol pro Mol der Grignard-Verbindung der allgemeinen Formel (III), bevorzugt zwischen 1 und 1,5 Mol pro Mol.

Als Lösungs- und Verdünnungsmittel im zweiten Schritt des erfindungsgemäßen Verfahrens wird naturgemäß dasjenige verwendet, das im ersten Schritt benutzt wurde.

Die Reaktionstemperatur liegt zwischen 10 und 70°C, bevorzugt zwischen 10 und 40°C.

Das Produkt des zweiten Schrittes wird nicht isoliert sondern als Lösung oder Suspension in den dritten Schritt eingesetzt.

### Dritter Schritt (3) des erfindungsgemäßen Verfahrens:

Als Lösungs- und Verdünnungsmittel im dritten Schritt des erfindungsgemäßen Verfahrens wird naturgemäß dasjenige verwendet, das im ersten und zweiten Schritt benutzt wurde.

Zur Hydrolyse der Verbindung der allgemeinen Formel (V) können verschiedene Säuren im Gemisch mit Wasser verwendet werden; beispielsweise: Salzsäure, Schwefelsäure, Phosphorsäure, Zitronensäure oder Essigsäure. Bevorzugt verwendet man Salzsäure oder Schwefelsäure.

Die Reaktionstemperatur liegt zwischen 10 und 70°C, bevorzugt zwischen 20 und 50°C.

Die Aufarbeitung erfolgt nach bekannten Methoden der organischen Chemie wie Filtration, Phasentrennung, Extraktion und Destillation.

### Vierter Schritt (4) des erfindungsgemäßen Verfahrens:

Als Lösungs- und Verdünnungsmittel im vierten Schritt des erfindungsgemäßen Verfahrens kommen beispielsweise in Frage: Ether wie beispielsweise Methyl-tert-butyl-ether, Cyclopentyl-methyl-ether, Tert-Amyl-methylether, 1,2-Dimethoxyethan, Diethylenglykol-diethylether, Tetrahydrofuran, 2-Methyl-tetrahydrofuran, 1,4-Dioxan; Nitrile wie beispielsweise Acetonitril oder Butyronitril; Ester wie beispielsweise Essigsäuremethylester oder Essigsäurebutylester; Kohlenwasserstoffe wie beispielsweise Hexan, Methylcyclohexan, Heptan, Toluol, o-Xylol, m-Xylol, p-Xylol, Mesitylen oder Chlorbenzol; Alkohole wie beispielsweise Methanol, Ethanol, Propanol, Isopropanol oder Butanol; oder Mischungen dieser Lösungs- und Verdünnungsmittel.

Es ist auch möglich, die Hydrierung bei Temperaturen oberhalb der Schmelzpunkte der Verbindungen der allgemeinen Formeln (VI) und (VII) ohne Gegenwart eines Lösungs- oder Verdünnungsmittels durchzuführen.

Bevorzugt als Lösungs- und Verdünnungsmittel sind Methanol, Ethanol, Tetrahydrofuran, 2-Methyl-tetrahydrofuran, Essigsäuremethylester oder Mischungen dieser Lösungs- und Verdünnungsmittel, sowie die Durchführung bei Temperaturen oberhalb der Schmelzpunkte der Verbindungen der allgemeinen Formeln (VI) und (VII) ohne Gegenwart eines Lösungs- oder Verdünnungsmittels.

Als Katalysatoren im vierten Schritt des erfindungsgemäßen Verfahrens kommen grundsätzlich all diejenigen in Betracht, die zur Hydrierung eines Benzaldehyds zum entsprechenden Benzylalkohol geeignet sind, beispielsweise Katalysatoren mit den Metallen Palladium, Platin, Iridium, Rhodium, Ruthenium, Cobalt oder Nickel. Bevorzugt sind Katalysatoren mit dem Metall Ruthenium, Cobalt oder Nickel.

Die Hydrierung kann sowohl mit homogen gelösten als auch mit heterogenen Katalysatoren durchgeführt werden. Beispielhaft seien genannt: Cobalt-Schwamm-Katalysator (Raney-Cobalt), Nickel-Schwamm-Katalysator (Raney-Nickel), Palladium auf Kohle, Platin auf Kohle, Ruthenium auf Kohle, {Bis[2-(diphenylphosphino)ethyl]amine}carbonylchlorohydridoruthenium(II) (Ru-MACHO, CAS 1295649-40-9), Dichlorotriphenylphosphine[bis(2-(ethylthio)ethyl)amine]ruthenium (II) (CAS 1462397-86-9), [2-(Aminomethyl)pyridine](dichloro)(diphenylphosphinobutane)ruthenium (II) (CAS 850424-32-7), Chloro[N-[(1R,2R)-1,2-diphenyl-2-[[3-(η6-phenyl)propyl]amino-κN]ethyl]-4-methylbenzenesulfon-amidato-κN]ruthenium (CAS 1192620-83-9).

Die Reaktionstemperatur liegt zwischen 20 und 200°C, bevorzugt zwischen 50 und 150°C.

Die Hydrierung kann bei Normaldruck oder unter erhöhtem Druck durchgeführt werden. Bevorzugt arbeitet man unter erhöhtem Druck von 1 bis 100 bar Wasserstoff, besonders bevorzugt von 10 bis 50 bar Wasserstoff.

Die Aufarbeitung erfolgt nach bekannten Methoden der organischen Chemie wie Filtration, Phasentrennung, Extraktion und Destillation.

### Fünfter Schritt (5) des erfindungsgemäßen Verfahrens:

Als Lösungs- und Verdünnungsmittel im fünften Schritt des erfindungsgemäßen Verfahrens kommen beispielsweise in Frage: Ether wie beispielsweise Methyl-tert-butyl-ether, Cyclopentyl-methyl-ether, Tert-Amyl-methylether, 1,2-Dimethoxyethan, Diethylenglykol-diethylether, Tetrahydrofuran, 2-Methyl-tetrahydrofuran, 1,4-Dioxan; Nitrile wie beispieslweise Acetonitril oder Butyronitril; Ester wie beispielsweise Essigsäuremethylester oder Essigsäurebutylester; Amide wie beispielsweise N,N-Dimethylformamid, N,N-Dimethylacetamid oder N-Methyl-pyrrolidon; Kohlenwasserstoffe wie beispielsweise Hexan, Methyl-cyclohexan, Heptan, Toluol, o-Xylol, m-Xylol, p-Xylol, Mesitylen oder Chlorbenzol oder Mischungen dieser Lösungs- und Verdünnungsmittel. Bevorzugt sind Methyl-cyclohexan, Heptan, Toluol, Xylol oder Chlorbenzol oder Gemische dieser Lösungs- und Verdünnungsmittel.

Verbindungen der allgemeinen Formel (VIII) mit Y gleich Brom können nach prinzipiell bekannten Methoden der organischen Chemie durch Reaktion der Verbindungen der allgemeinen Formel (VII) mit Bromierungsmitteln wie beispielsweise Bromwasserstoff, N-Brom-succinimid, Phosphortribromid oder Thionylbromid erhalten werden. Bevorzugt verwendet man Bromwasserstoff oder Thionylbromid.

Verbindungen der allgemeinen Formel (VIII) mit Y gleich Chlor können nach prinzipiell bekannten Methoden der organischen Chemie durch Reaktion der Verbindungen der allgemeinen Formel (VII) mit Chlorierungsmitteln wie beispielsweise Chlorwasserstoff, N-Chlor-succinimid, Phosphortrichlorid, Cyanursäuretrichlorid, Phosgen oder Thionylchlorid erhalten werden. Bevorzugt verwendet man Chlorwasserstoff, Phosgen oder Thionylchlorid.

Bei der Herstellung der Verbindungen der allgemeinen Formel (VIII) mit Y gleich Chlor unter Verwendung von Chlorwasserstoff, Phosgen oder Thionylchlorid ist es zur Erzielung einer hohen Ausbeute bevorzugt, das Chlorierungsmittel vorzulegen und den Benzylalkohol der allgemeinen Formel (VII) zu diesem zu dosieren. Besonders bevorzugt ist es, Thionylchlorid vorzulegen und den Benzylalkohol der allgemeinen Formel (VII) zu diesem zu dosieren.

Verbindungen der allgemeinen Formel (VIII) mit Y gleich OSO₂Me, OSO₂(4-Methylphenyl) oder OSO₂CF₃ können nach prinzipiell bekannten Methoden der organischen Chemie durch Reaktion der Verbindungen der allgemeinen Formel (VII) mit den entsprechenden Sulfonsäurechloriden oder Sulfonsäureanhydriden hergestellt werden.

### Sechster Schritt (6) des erfindungsgemäßen Verfahrens:

Als Lösungs- und Verdünnungsmittel im sechsten Schritt des erfindungsgemäßen Verfahrens kommen beispielsweise in Frage: Ether wie beispielsweise Methyl-tert-butyl-ether, Cyclopentyl-methyl-ether, Tert-Amyl-methylether, 1,2-Dimethoxyethan, Diethylenglykol-diethylether, Tetrahydrofuran, 2-Methyl-tetrahydrofuran, 1,4-Dioxan; Nitrile wie beispieslweise Acetonitril oder Butyronitril; Ester wie beispielsweise Essigsäuremethylester oder Essigsäurebutylester; Amide wie beispielsweise N,N-Dimethylformamid, N,N-Dimethylacetamid oder N-Methyl-pyrrolidon; Kohlenwasserstoffe wie beispielsweise Hexan, Methyl-cyclohexan, Heptan, Toluol, o-Xylol, m-Xylol, p-Xylol, Mesitylen oder Chlorbenzol; Wasser oder Mischungen dieser Lösungs- und Verdünnungsmittel. Bevorzugt sind Methyl-cyclohexan, Heptan, Toluol, o-Xylol, m-Xylol, p-Xylol, Chlorbenzol oder Wasser oder Gemische dieser Lösungs- und Verdünnungsmittel.

Als Cyanierungsmittel der allgemeinen Formel (IX) können Lithiumcyanid, Natriumcyanid oder Kaliumcyanid eingesetzt werden. Bevorzugt verwendet man Natriumcyanid oder Kaliumcyanid.

Die Menge an eingesetztem Cyanid liegt zwischen 0,9 und 2 Mol pro Mol Verbindung der allgemeinen Formel (VIII), bevorzugt zwischen 1 und 1,5 Mol pro Mol.

Wenn die Reaktion in einem Zwei-Phasen-Gemisch von Lösungs- und Verdünnungsmitteln stattfindet, wird sie in der Regel in Gegenwart eines Phasentransferkatalysators durchgeführt. Solche Phasentransferkatalysatoren können beispielsweise Tetra-alkyl-ammoniumsalze sein, wie beispielsweise Tetrabutylammoniumbromid, Tetraoctylammoniumchlorid oder Tetradecylammoniumchlorid, oder Gemische solcher Tetra-alkyl-ammoniumsalze wie beispielsweise Aliquat336.

Die Menge an Phasentransferkatalysator liegt zwischen 0,01 und 10 Molprozent, bezogen auf Verbindung der allgemeinen Formel (VIII), bevorzugt zwischen 0,1 und 5 Molprozent.

Die Reaktionstemperatur liegt zwischen 20 und 200°C, bevorzugt zwischen 50 und 150°C.

Die Reaktion kann auch unter vermindertem oder erhöhtem Druck durchgeführt werden.

Die Aufarbeitung erfolgt nach bekannten Methoden der organischen Chemie wie Filtration, Phasentrennung, Extraktion und Destillation.

### Siebter Schritt (7) des erfindungsgemäßen Verfahrens:

Als Lösungs- und Verdünnungsmittel im siebten Schritt des erfindungsgemäßen Verfahrens kommen beispielsweise in Frage: Kohlenwasserstoffe wie beispielsweise Hexan, Methyl-cyclohexan, Heptan, Toluol, o-Xylol, m-Xylol, p-Xylol, Mesitylen oder Chlorbenzol; Alkohole wie Methanol, Ethanol, n-Propanol, Isopropanol, Butanol, Ethylenglykol, Diethylenglykol, Triethylenglykol; Wasser oder Mischungen dieser Lösungs- und Verdünnungsmittel. Bevorzugt sind Methanol, Ethanol, n-Propanol, Isopropanol, Butanol, Ethylenglykol, Toluol, o-Xylol, m-Xylol, p-Xylol, Mesitylen oder Wasser oder Gemische dieser Lösungs- und Verdünnungsmittel.

Wenn die Reaktion in einem Zwei-Phasen-Gemisch von Lösungs- und Verdünnungsmitteln stattfindet, wird sie in der Regel in Gegenwart eines Phasentransferkatalysators durchgeführt. Solche Phasentransferkatalysatoren können beispielsweise Tetra-alkyl-ammoniumsalze sein, wie beispielsweise Tetrabutylammoniumbromid, Tetraoctylammoniumchlorid oder Tetradecylammoniumchlorid, oder Gemische solcher Tetra-alkyl-ammoniumsalze wie beispielsweise Aliquat336.

Der siebte Schritt des erfindungsgemäßen Verfahrens kann grundsätzlich unter sauren oder alkalischen Bedingungen durchgeführt werden.

Für die Durchführung unter sauren Bedingungen verwendet man Säuren wie beispielsweise Salzsäure, Schwefelsäure oder Phosphorsäure im Gemisch mit Wasser. Bevorzugt verwendet man Schwefelsäure im Gemisch mit Wasser.

Für die Durchführung unter alkalischen Bedingungen verwendet man Basen wie beispielsweise Lithiumhydroxid, Natriumhydroxid, Kaliumhydroxid oder Calciumhydroxid. Bevorzugt verwendet man Natriumhydroxid oder Kaliumhydroxid.

Die Reaktionstemperaturen liegen zwischen 50 und 250°C, bevorzugt zwischen 80 und 200°C.

Die Reaktion kann auch unter vermindertem oder erhöhtem Druck durchgeführt werden.

Die Aufarbeitung erfolgt nach bekannten Methoden der organischen Chemie wie Filtration, Phasentrennung, Extraktion und Destillation.

Die vorliegende Erfindung soll durch die folgenden Beispiele näher erläutert werden, ohne dass sie dadurch eingeschränkt werden soll.

### Beispiele

### Beispiel 1: Brom(4-chlor-2,6-dimethylphenyl)magnesium

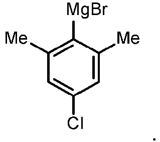

Man legt in einem 250mL-Dreihalskolben unter Argon 2,67 g [109,9 mMol] Magnesiumspäne und ein Kriställchen Iod vor. Unter Rühren wird der Kolbeninhalt mit einem Heißluftfön erhitzt bis Ioddämpfe sichtbar sind. Man fügt etwa 10 mL einer Lösung von 21,7 g [99 mMol] 4-Chlor-2,6-dimethyl-brombenzol in 100 mL Tetrahydrofuran (THF) hinzu und erwärmt auf 50°C, bis ein Anspringen der Reaktion erkennbar wird. Dann werden die restliche Eduktlösung langsam zudosiert, wobei die Innentemperatur durch Kühlung bei 50°C gehalten wird. Abschließend rührt man noch für eine Stunde nach.

### Beispiel 2: Brom(4-chlor-2,6-dimethylphenyl)magnesium

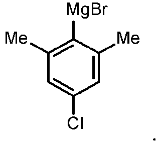

In einem 2L-Doppelmantelgefäß werden unter Argon 20,05 g [0,825 Mol] Magnesiumspäne vorgelegt. Man gibt bei 25°C zuerst 50 mL der Lösung aus Beispiel 1 hinzu und dann 25 g einer Lösung von 164,6 g [0,75 Mol] 4-Chlor-2,6-dimethyl-brombenzol in 565 mL THF. Das Anspringen der Reaktion wird durch die Exothermie deutlich. Dann wird die restliche Menge der Eduktlösung innerhalb von etwa 2,5 Stunden so zudosiert, dass die Innentemperatur 33°C nicht überschreitet. Am Ende wird noch für eine Stunde bei 35°C nachgerührt. Verrühren einer kleinen Ansatzprobe in einer THF-Lösung von Iod und anschließende HPLC-Analyse zeigt einen vollständigen Umsatz des 4-Chlor-2,6-dimethyl-brombenzol an.

### Beispiel 3: Magnesiumbromid-(4-chlor-2,6-dimethylphenyl)(dimethylamino)methanolat

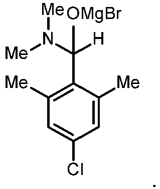

In einem 2L-Doppelmantelgefäß wird bei 27 - 35°C innerhalb von etwa einer Stunde zu der Lösung aus Beispiel 2 eine Lösung von 54,8 g [0,75 Mol] N,N-Dimethylformamid (DMF) in 185 mL THF dosiert. Anschließend rührt man noch für eine Stunde bei 27 - 35°C. Das erhaltene Produkt wird ohne weitere Aufarbeitung in die nächste Stufe eingesetzt.

### Beispiel 4: 4-Chlor-2,6-dimethylbenzaldehyd

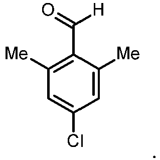

In einem 2L-Doppelmantelgefäß werden bei 15°C 402 g halbkonzentrierte Salzsäure zu dem Reaktionsgemisch aus Beispiel 3 dosiert, so dass der pH-Wert auf 1 fällt. Man lässt das Reaktionsgemisch ab, trennt die Phasen, extrahiert die wässrige Phase dreimal mit je 200 mL Methyl-tert-butyl-ether (MTBE), wäscht die vereinigten organischen Phasen mit 100 mL gesättigter wässriger Natriumchloridlösung, trocknet über Natriumsulfat und engt unter vermindertem Druck ein. Es resultieren 131,5 g gelblicher Feststoff. Nach Entfernung leichtsiedender Verunreinigungen bei 100°C und 6 mbar verbleiben 123,4 g gelblicher Feststoff, der nach GC-Analyse 90,7% der Titelverbindung enthält, womit sich eine Ausbeute von 83% der Theorie, bezogen auf das Ausgangsmaterial in Beispiel 2, ergibt.

GC/MS: m/e = 167 ((M-1)⁺, ³⁵Cl, 100%), 139 (M-29, 45%).

¹H-NMR (600 MHz, CDCl₃): δ = 2,59 (s, 6H), 7,09 (s, 2H), 10,55 (s, 1H) ppm.
Schmelzpunkt: 59°C

### Beispiel 5: 4-Chlor-2,6-dimethylbenzylalkohol

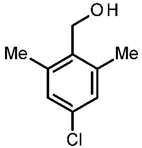

In einem 2L-Autoklav werden 120,8 g 4-Chlor-2,6-dimethylbenzaldehyd mit einer Reinheit von 89,2% [0,639 Mol] in 480 mL Ethanol vorgelegt. Man gibt 3,6 g Raney-Cobalt (Actimet; jeweils dreimal mit Wasser und Ethanol gewaschen) hinzu, verschließt den Autoklaven, spült zweimal mit Argon und hydriert dann 16 Stunden bei 100°C und 30 bar Wasserstoffdruck. Nach Abkühlen auf Raumtemperatur und Entspannen wird das Reaktionsgemisch über Celite filtriert und das Filtrat im Vakuum eingeengt. Man erhält 115,6 g Produkt, das nach quantitativem ¹H-NMR zu 90,4% aus der Titelverbindung besteht, womit sich eine Ausbeute von 95,8% der Theorie ergibt.

GC/MS: m/e = 170 (M⁺, ³⁵Cl, 35%), 152 (M-18, ³⁵Cl, 100%).

¹H-NMR (600 MHz, d₆-DMSO): δ = 2,34 (s, 6H), 4,44 (d, J = 5,3 Hz, 2H), 4,75 (t, J = 5,3 Hz, 1H), 7,06 (s, 2H) ppm.
Schmelzpunkt (auf 98,3% aufgereinigte Verbindung): 110,6°C

### Beispiel 6: 4-Chlor-2,6-dimethylbenzylalkohol

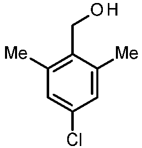

Eine Lösung von 1 g 4-Chlor-2,6-dimethylbenzaldehyd mit einer Reinheit von 94% in 10 ml Tetrahydrofuran wird in einen Autoklaven gegeben und mit 8 mg [2-(Aminomethyl)pyridin](dichloro)-(diphenylphosphinobutan)ruthenium (II) (CAS 850424-32-7) und 16 µl einer 1,7 M Lösung von Kaliumtert-butylat in THF versetzt. Der Autoklav wird zweimal mit 10 bar Argon gespült und dann 18 Stunden bei 50°C mit 50 bar Wasserstoff beaufschlagt. Nach Abkühlen auf Raumtemperatur und Entspannen wird die Titelverbindung nach GC/MS-Analyse mit einer Reinheit von 91,7% erhalten.

GC/MS: m/e = 170 (M⁺, ³⁵Cl, 35%), 152 (M-18, ³⁵Cl, 100%).

### Beispiel 7: 2-(Brommethyl)-5-chlor-1,3-dimethylbenzol

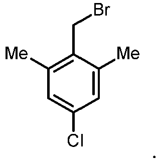

Man erhitzt 8,53 g [50 mMol] 4-Chlor-2,6-dimethylbenzylalkohol in 60 mL 48%iger wässriger Bromwasserstoffsäure für 4 Stunden auf 92°C. Das Reaktionsgemisch wird auf Raumtemperatur gekühlt und mit 50 mL Methylenchlorid versetzt. Die Phasen werden getrennt und die wässrige Phase zweimal mit je 50 mL Methylenchlorid extrahiert. Die vereinigten organischen Phasen werden mit 50 mL Wasser und dann 30 mL gesättigter Natriumbicarbonatlösung ausgeschüttelt, über Natriumsulfat getrocknet und im Vakuum eingeengt. Man erhält 12,22 g Feststoff, der nach GC/MS-Analyse 91,1% der Titelverbindung enthält, womit sich eine Ausbeute von 95,3% der Theorie ergibt.

GC/MS: m/e = 232 (M⁺, ³⁵Cl, ⁷⁹Br, 5%), 153 (M-79, 100%).

### Beispiel 8: 2-(Chlormethyl)-5-chlor-1,3-dimethylbenzol

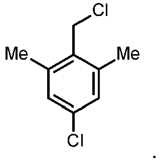

Man legt 14,94 g [0,19 Mol] Thionylchlorid vor, erwärmt es auf 72°C und tropft innerhalb einer Stunde eine warme Lösung (74°C) von 16,47 g [0,0965 Mol] 4-Chlor-2,6-dimethylbenzylalkohol in 75 mL Toluol zu. Anschließend rührt man noch für 90 Minuten bei 72°C. Der Überschuß an Thionylchlorid wird abdestilliert, der Rückstand über etwas Celite filtriert und im Vakuum eingeengt. Man erhält 20,21 g eines grünlichen Feststoffes, der nach GC/MS-Analyse 87,2% der Titelverbindung enthält, womit sich eine Ausbeute von 96,5% der Theorie ergibt.

GC/MS: m/e = 188 (M⁺, ³⁵Cl, 12%), 153 (M-35, 100%), 119 (M-36, 72%).

¹H-NMR (600 MHz, CDCl₃): δ = 2,33 (s, 6H), 4,53 (s, 2H), 6,97 (s, 2H) ppm.
Schmelzpunkt: 63,5 - 64°C

### Beispiel 9: (4-Chlor-2,6-dimethylphenyl)acetonitril

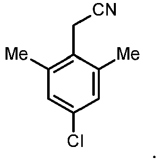

Man legt eine Lösung von 70,8 g 2-(Chlormethyl)-5-chlor-1,3-dimethylbenzol einer Reinheit von 74,4% in 105 mL Toluol vor, gibt 35 mL Wasser und 1,13 g Aliquat336 hinzu, erwärmt auf 65°C und dosiert unter gutem Rühren eine Lösung von 16,38 g [0,334 Mol] Natriumcyanid in 55 mL Wasser zu. Anschließend rührt man 16 Stunden bei 80°C. Man trennt die Phasen bei Raumtemperatur, wäscht die organische Phase mit 120 mL gesättigter wässriger Natriumbicarbonatlösung und zweimal 100 mL Wasser, trocknet über Natriumsulfat und engt im Vakuum ein. Es resultieren 61,9 g Feststoff, der nach quantitativem ¹H-NMR 69,8% der Titelverbindung enthält, womit sich eine Ausbeute von 86,3% der Theorie ergibt. Durch Umkristallisation aus 100 ml Isopropanol erhält man 33,1 g Feststoff, der nach GC/MS-Analyse 99,2% der Titelverbindung enthält, womit sich eine Ausbeute von 65,6% der Theorie ergibt.

GC/MS: m/e = 179 (M⁺, ³⁵Cl, 57%), 152 (M-27, 100%), 144 (70%), 118 (90%).

¹H-NMR (600 MHz, d6-DMSO): δ = 2,34 (s, 6H), 3,89 (s, 2H), 7,2 (s, 2H) ppm.
Schmelzpunkt: 87,6°C

### Beispiel 10: (4-Chlor-2,6-dimethylphenyl)essigsäure

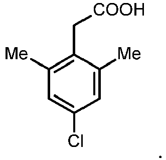

Man legt 28,1 g (4-Chlor-2,6-dimethylphenyl)acetonitril einer Reinheit von 82% und 45,6 g (4-Chlor-2,6-dimethylphenyl)acetonitril einer Reinheit von 84,8% in 300 mL Ethanol vor, gibt 122 g 45%iger Natronlauge hinzu und rührt 48 Stunden unter Rückfluß. Nach Abkühlen auf Raumtemperatur gibt man das Reaktionsgemisch auf Eis, stellt mitt konzentrierte Salzsäure auf einen pH-Wert von 1, saugt den Feststoff ab, wäscht ihn mit Wasser und trocknet ihn. Man erhält so 76,04 g Feststoff, der nach quantitativem ¹H-NMR eine Reinheit von 86,8% aufweist, womit sich eine Ausbeute von 96,8% der Theorie ergibt.

GC/MS: m/e = 198 (M⁺, ³⁵Cl, 23%), 153 (M-45, 100%), 115 (23%).

¹H-NMR (600 MHz, d6-DMSO): δ = 2,34 (s, 6H), 3,58 (s, 2H), 7,1 (s, 2H) ppm.
Schmelzpunkt (nach Umkristallisation): 188,7°C

### Beispiel 11: Brom(2,6-dimethylphenyl)magnesium (nicht erfindungsgemäß)

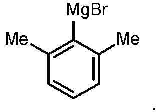

Man legt in einem 250mL-Dreihalskolben unter Argon 4,01 g [165 mMol] Magnesiumspäne und ein Kriställchen Iod vor. Unter Rühren wird der Kolbeninhalt mit einem Heißluftfön erhitzt bis Ioddämpfe sichtbar sind. Man fügt etwa 10 mL einer Lösung von 27,76 g [150 mMol] 2,6-Dimethyl-brombenzol in 150 mL Tetrahydrofuran (THF) hinzu und erwärmt auf 50°C, bis ein Anspringen der Reaktion erkennbar wird. Dann werden die restliche Eduktlösung langsam zudosiert, wobei die Innentemperatur durch Kühlung bei 50°C gehalten wird. Abschließend rührt man noch für eine Stunde nach.

### Beispiel 12: Brom(2,6-dimethylphenyl)magnesium (nicht erfindungsgemäß)

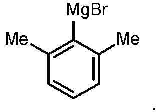

In einem 6L-Doppelmantelgefäß werden unter Argon 66,62 g [2,741 Mol] Magnesiumspäne vorgelegt. Man gibt bei 25°C zuerst die Lösung aus Beispiel 11 hinzu und dann 800 mL THF. Bei 30°C werden dann 100 g einer Lösung von 461,2 g [2,492 Mol] 2,6-Dimethyl-brombenzol in 1200 mL THF zudosiert. Das Anspringen der Reaktion wird durch die Exothermie deutlich. Dann wird die restliche Menge der Eduktlösung innerhalb von 100 Minuten so zudosiert, dass die Innentemperatur 33°C nicht überschreitet. Am Ende wird noch für zwei Stunden bei 35°C nachgerührt. Verrühren einer kleinen Ansatzprobe in einer THF-Lösung von Iod und anschließende HPLC-Analyse zeigt einen vollständigen Umsatz des 2,6-Dimethyl-brombenzol an.

### Beispiel 13: Magnesiumbromid-(2,6-dimethylphenyl)(dimethylamino)methanolat (nicht erfindungsgemäß)

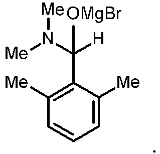

In einem 6L-Doppelmantelgefäß wird bei 24 - 29°C innerhalb von etwa 90 Minuten zu der Lösung aus Beispiel 12 eine Lösung von 193,1 g [2,642 Mol] DMF in 500 mL THF dosiert. Anschließend rührt man noch für eine Stunde bei 27°C. Das erhaltene Produkt wird ohne weitere Aufarbeitung in die nächste Stufe eingesetzt.

### Beispiel 14: 2,6-Dimethylbenzaldehyd (nicht erfindungsgemäß)

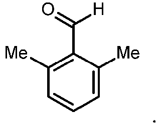

In einem 6L-Doppelmantelgefäß werden bei 15 - 20°C 1500 g halbkonzentrierte Salzsäure zu dem Reaktionsgemisch aus Beispiel 13 dosiert, so dass der pH-Wert auf 1 fällt, und rührt noch drei Stunden bei Raumtemperatur. Man lässt das Reaktionsgemisch ab, trennt die Phasen, extrahiert die wässrige Phase zweimal mit je 500 mL MTBE, wäscht die vereinigten organischen Phasen mit 500 ml gesättigter wässriger Natriumchloridlösung, trocknet über Natriumsulfat und engt unter vermindertem Druck ein. Es resultieren 318,7 g gelblicher Feststoff, der nach quantitativen ¹H-NMR 84,0% der Titelverbindung enthält, womit sich eine Ausbeute von 75,5% der Theorie, bezogen auf das Ausgangsmaterial in Beispiel 12, ergibt.

GC/MS: m/e = 133 ((M-1)⁺, ³⁵Cl, 100%), 105 (M-29, 100%).

¹H-NMR (600 MHz, CDCl₃): δ = 2,55 (s, 6H), 7,15 (m, 2H), 7,39 (m, 1H), 10,53 (s, 1H) ppm.

### Beispiel 15: 2,6-Dimethylbenzylalkohol (nicht erfindungsgemäß)

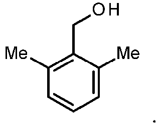

In einem 5L-Autoklaven werden 318,7 g der Verbindung aus Beispiel 14 als Lösung in 1300 mL Ethanol vorgelegt. Man gibt 3,9 g Raney-Cobalt (Actimet), das zweimal mit Wasser und dreimal mit Ethanol gewaschen wurde, hinzu, spült den verschlossenen Autoklaven zweimal mit Argon und hydriert dann für 34 Stunden bei 100°C und 30 bar Wasserstoffdruck. Das Reaktionsgemisch wird anschließend über Celite filtriert und dann im Vakuum eingeengt. Man erhält 299,6 g Feststoff, der nach GC/MS-Analyse 74,7% der Titelverbindung enthält, womit sich eine Ausbeute von 74% der Theorie ergibt.

GC/MS: m/e = 138 (M⁺, 20%), 118 (100%).

### Beispiel 16: 2-(Chlormethyl)-1,3-dimethylbenzol (nicht erfindungsgemäß)

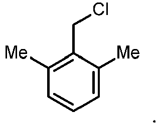

Man legt 91,4 g [0,768 Mol] Thionylchlorid in 100 mL Toluol vor, erwärmt es auf 72°C und tropft innerhalb einer Stunde eine Lösung von 100 g 2,6-Dimethylbenzylalkohol einer Reinheit von 74,7% in 700 mL Toluol zu. Anschließend rührt man noch für eine Stunde bei 72°C. Der Überschuß an Thionylchlorid wird abdestilliert, der Rückstand über etwas Celite filtriert und im Vakuum eingeengt. Man erhält 107,2 g eines braunen Öls, das nach GC/MS-Analyse 71,0% der Titelverbindung enthält, womit sich eine Ausbeute von 89,7% der Theorie ergibt.

GC/MS: m/e = 154 (M⁺, ³⁵Cl, 17%), 119 (M-35, 100%),

### Beispiel 17: 2,6-Dimethylphenyl-acetonitril (nicht erfindungsgemäß)

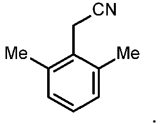

Man legt eine Lösung von 105,6 g 2-(Chlormethyl)-1,3-dimethylbenzol einer Reinheit von 68,9% in 150 mL Toluol vor, gibt 50 mL Wasser und 1,9 g Aliquat336 hinzu, erwärmt auf 65°C und dosiert unter gutem Rühren eine Lösung von 27,68 g [0,565 Mol] Natriumcyanid in 80 mL Wasser zu. Anschließend rührt man 16 Stunden bei 80°C. Man setzt danach weitere 0,85 g Aliquat336 und 2,3 g Natriumcyanid zu und rührt 18 Stunden bei 80°C. Man trennt die Phasen bei Raumtemperatur, wäscht die organische Phase mit 120 mL gesättigter wässriger Natriumbicarbonatlösung und zweimal 100 mL Wasser, trocknet über Natriumsulfat und engt im Vakuum ein. Es resultieren 85 g Rohprodukt, das nach GC/MS-Analyse 79,3% der Titelverbindung enthält, womit sich eine Ausbeute von 98,6% der Theorie ergibt.

GC/MS: m/e = 145 (M⁺, 40%), 118 (M-27, 100%).

### Beispiel 18: 2,6-Dimethylphenyl-essigsäure (nicht erfindungsgemäß)

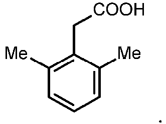

In einer Mischung aus 100 mL Triethylenglykol und 25 mL Wasser werden 11 g 2,6-Dimethylphenyl-acetonitril einer Reinheit von 94% vorgelegt. Man gibt 28,1 g KOH-Plätzchen (85%ig) zu und rührt 18 Stunden bei 120°C. Man läßt auf 50°C abkühlen, rührt das Reaktionsgemisch dann in 500 mL eiskaltes Wasser ein, stellt mit 32%iger Salzsäure auf einen pH-Wert von 1, filtriert den Feststoff ab, wäscht ihn zweimal mit je 75 mL Wasser und trocknet ihn. Es werden 9,89 Feststoff erhalten, der laut HPLC-Analyse 96,3% der Titelverbindung enthält, womit sich eine Ausbeute von 81,7% der Theorie ergibt.

GC/MS(sil.): m/e = 236 (M⁺(sil.), 7%), 221 (M⁺(sil.)-15, 10%), 192 (10%), 119 (13%), 73(100%).

## Patentansprüche

1. Verfahren zur Herstellung von Verbindungen der Formel (I) 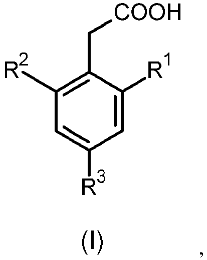 in welcher
R¹ und R² für Methyl stehen
und
R³ für Chlor steht,
**dadurch gekennzeichnet, dass** man in einem ersten Schritt (1) Verbindungen der Formel (II) 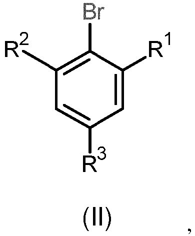
in der R¹, R² und R³ die oben angegebenen Bedeutungen haben,
in Gegenwart eines Lösungsmittels mit Magnesium zu Verbindungen der Formel (III) umsetzt, 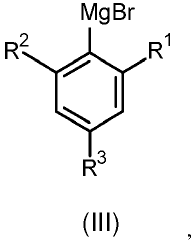
in der R¹, R² und R³ die oben angegebenen Bedeutungen haben;
in einem zweiten Schritt (2) die Verbindungen der Formel (III) mit Verbindungen der Formel (IV) 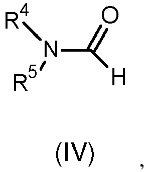
in der
R⁴ und R⁵ für Methyl stehen,
zu einer Verbindung der Formel (V) umsetzt 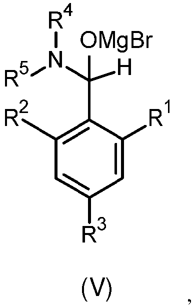
in der R¹, R², R³, R⁴ und R⁵ die oben angegebenen Bedeutungen haben;
in einem dritten Schritt (3) die Verbindungen der Formel (V) durch Hydrolyse unter sauren Bedingungen zu Verbindungen der Formel (VI) 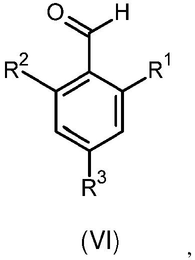
in der R¹, R² und R³ die oben angegebenen Bedeutungen haben,
umsetzt;
in einem vierten Schritt (4) Verbindungen der Formel (VI) zu Verbindungen der Formel (VII) 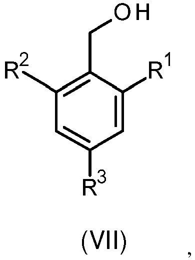
in der R¹, R² und R³ die oben angegebenen Bedeutungen haben,
in Gegenwart eines Katalysators hydriert;
in einem fünften Schritt (5) Verbindungen der Formel (VII) zu Verbindungen der Formel (VIII) 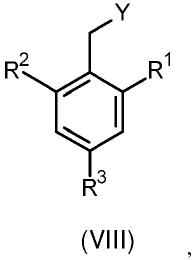
in der R¹, R² und R³ die oben angegebenen Bedeutungen haben und
Y für Chlor oder Brom steht,
umsetzt;
in einem sechsten Schritt (6) die Verbindungen der Formel (VIII) mit einem Cyanid der Formel (IX)
MCN (IX),
in der
M für Natrium steht,
zu Verbindungen der Formel (X) 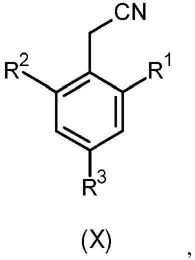
in der R¹, R² und R³ die oben angegebenen Bedeutungen haben,
umsetzt;
in einem siebten Schritt (7) die Verbindungen der Formel (X) unter sauren oder basischen Bedingungen zu Verbindungen der Formel (I) 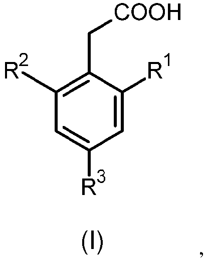
in der R¹, R² und R³ die oben angegebenen Bedeutungen haben,
hydrolysiert.

2. Verfahren gemäß Anspruch 1, wobei für die Hydrierung im vierten Schritt (4) Katalysatoren mit dem Metall Ruthenium, Cobalt oder Nickel zum Einsatz kommen.

3. Verfahren gemäß Anspruch 1, wobei für die Hydrierung im vierten Schritt (4) als Katalysator Raney-Cobalt zum Einsatz kommt.

4. Verfahren gemäß Anspruch 1, wobei für die Hydrierung im vierten Schritt (4) als Katalysator Raney-Nickel zum Einsatz kommt.

5. Verfahren gemäß Anspruch 1, wobei für die Hydrierung im vierten Schritt (4) als Katalysator [2-(Aminomethyl)pyridin](dichloro)(diphenylphosphinobutan)ruthenium (II) zum Einsatz kommt.

6. Verbindungen der Formel (V) 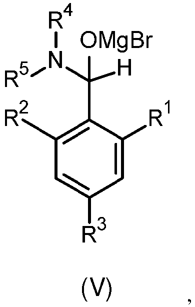 in welcher R¹, R², R³, R⁴ und R⁵ die oben angegebenen Bedeutungen haben.

## Claims

1. Process for preparing compounds of the formula (I) 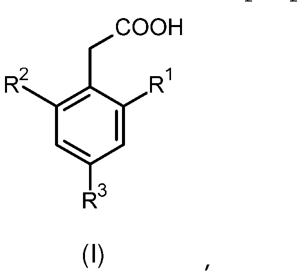 in which
R¹ and R² represent methyl
and
R³ represents chlorine,
**characterized in that** in a first step (1) compounds of the formula (II) 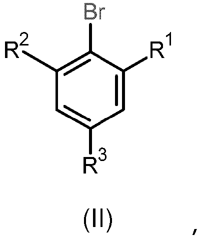
in which R¹, R² and R³ have the definitions given above, are reacted with magnesium in the presence of a solvent to give compounds of the formula (III), 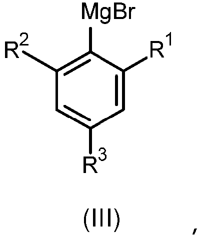
in which R¹, R² and R³ have the definitions given above; in a second step (2) the compounds of the formula (III) are reacted with compounds of the formula (IV) 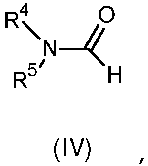
in which
R⁴ and R⁵ represent methyl,
to give a compound of the formula (V) 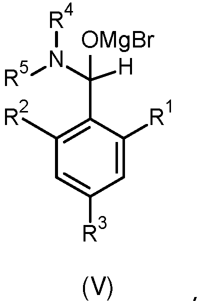
in which R¹, R², R³, R⁴ and R⁵ have the definitions given above;
in a third step (3) the compounds of the formula (V) are reacted by hydrolysis under acidic conditions to give compounds of the formula (VI) 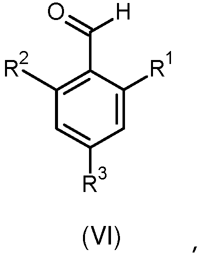
in which R¹, R² and R³ have the definitions given above;
in a fourth step (4) compounds of the formula (VI) are hydrogenated to give compounds of the formula (VII) 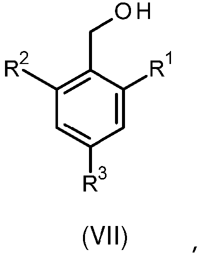
in which R¹, R² and R³ have the definitions given above; in the presence of a catalyst;
in a fifth step (5) compounds of the formula (VII) are reacted to give compounds of the formula (VIII) 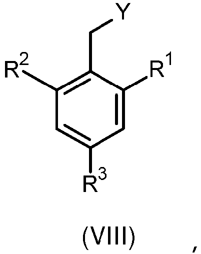
in which R¹, R² and R³ have the definitions given above and
Y represents chlorine or bromine;
in a sixth step (6) the compounds of the formula (VIII) are reacted with a cyanide of the formula (IX)
MCN (IX),
in which
M represents sodium,
to give compounds of the formula (X) 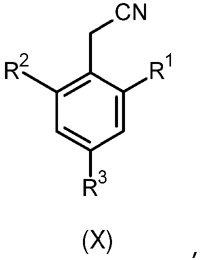
in which R¹, R² and R³ have the definitions given above;
in a seventh step (7) the compounds of the formula (X) are hydrolysed under acidic or basic conditions to give compounds of the formula (I) 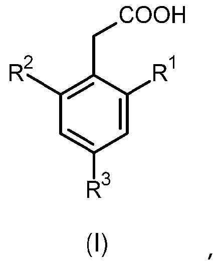
in which R¹, R² and R³ have the definitions given above.

2. Process according to Claim 1, wherein, for the hydrogenation in the fourth step (4), use is made of catalysts with the metal ruthenium, cobalt or nickel.

3. Process according to Claim 1, wherein, for the hydrogenation in the fourth step (4), Raney cobalt is used as catalyst.

4. Process according to Claim 1, wherein, for the hydrogenation in the fourth step (4), Raney nickel is used as catalyst.

5. Process according to Claim 1, wherein, for the hydrogenation in the fourth step (4), [2-(aminomethyl)pyridine] (dichloro) (diphenylphosphinobutan e)ruthenium (II) is used as catalyst.

6. Compounds of the formula (V) 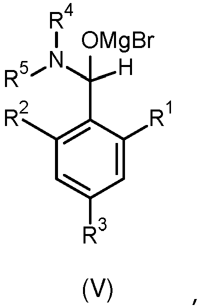 in which R¹, R², R³, R⁴ and R⁵ have the definitions given above.

## Revendications

1. Procédé de préparation de composés de formule (I) 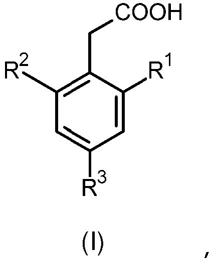 dans laquelle
R¹ et R² représentent un groupe méthyle
et
R³ représente le chlore,
**caractérisé en ce que**, dans une première étape (1), des composés de formule (II) 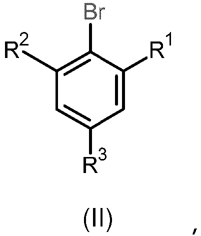
dans laquelle R¹, R² et R³ ont les significations indiquées plus haut,
sont mis en réaction en présence d'un solvant avec du magnésium pour aboutir à des composés de formule (III), 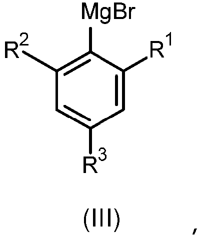
dans laquelle R¹, R² et R³ ont les significations indiquées plus haut,
dans une deuxième étape (2), les composés de formule (III) sont mis en réaction avec des composés de formule (IV) 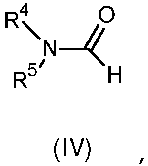
dans laquelle
R⁴ et R⁵ représentent un groupe méthyle ;
pour aboutir à un composé de formule (V) 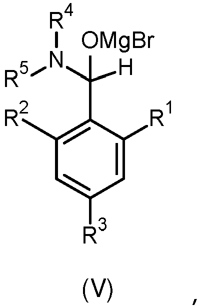
dans laquelle R¹, R², R³, R⁴ et R⁵ ont les significations indiquées ci-dessus ;
dans une troisième étape (3), les composés de formule (V) sont mis en réaction par hydrolyse dans des conditions acides pour aboutir à des composés de formule (VI) 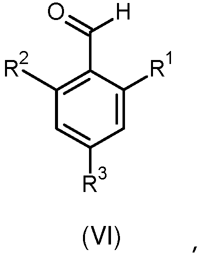
dans laquelle R¹, R² et R³ ont les significations indiquées plus haut ;
dans une quatrième étape (4), des composés de formule (VI) sont hydrogénés en composés de formule (VII) 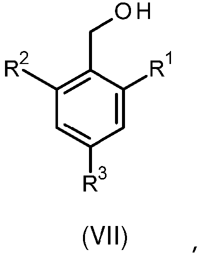
dans laquelle R¹, R² et R³ ont les significations indiquées plus haut ;
en présence d'un catalyseur ;
dans une cinquième étape (5), des composés de formule (VII) sont mis en réaction pour aboutir à des composés de formule (VIII) 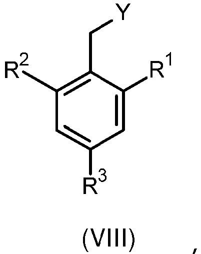
dans laquelle R¹, R² et R³ ont les significations indiquées plus haut et
Y représente le chlore ou le brome ;
dans une sixième étape (6), les composés de formule (VIII) sont mis en réaction avec un cyanure de formule (IX)
MCN (IX),
dans laquelle
M représente le sodium,
pour aboutir à des composés de formule (X') 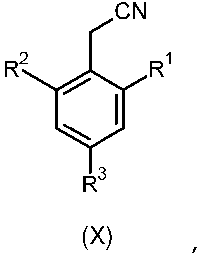
dans laquelle R¹, R² et R³ ont les significations indiquées plus haut ;
dans une septième étape (7), les composés de formule (X) sont hydrolysés dans des conditions acides ou basiques pour aboutir à des composés de formule (I) 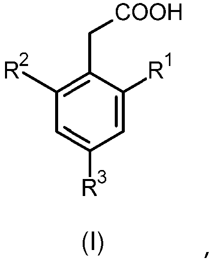
dans laquelle R¹, R² et R³ ont les significations indiquées plus haut.

2. Procédé selon la revendication 1, dans lequel des catalyseurs contenant le métal ruthénium, cobalt ou nickel sont utilisés pour l'hydrogénation dans la quatrième étape (4).

3. Procédé selon la revendication 1, dans lequel du cobalt de Raney est utilisé en tant que catalyseur pour l'hydrogénation dans la quatrième étape (4).

4. Procédé selon la revendication 1, dans lequel du nickel de Raney est utilisé en tant que catalyseur pour l'hydrogénation dans la quatrième étape (4).

5. Procédé selon la revendication 1, dans lequel du [2-(aminométhyl)pyridine] (dichloro) (diphénylphosphinobutan e)ruthénium (II) est utilisé en tant que catalyseur pour l'hydrogénation dans la quatrième étape (4).

6. Composés de formule (V) 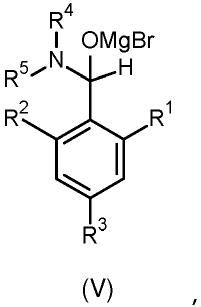 dans laquelle R¹, R², R³, R⁴ et R⁵ ont les significations indiquées plus haut.
